# EUROPEAN PATENT SPECIFICATION

(11) **EP 3 263 134 B1**
(45) Date of publication and mention of the grant of the patent: **05.05.2021**
(21) Application number: 15883426.7
(22) Date of filing: 27.02.2015
(51) Int. Cl.: A61K 31/4985, A61K 31/40, A61K 31/403, A61K 31/495, A61K 31/513, A61K 31/522, A61P 9/00, A23L 2/00, A61K 39/395, A61K 48/00, A23L 2/52, A61P 13/02, A61P 13/12, A61P 3/10, A61P 5/30, A61P 9/04

(54) **COMPOSITION FOR PREVENTING OR TREATING VALVE CALCIFICATION, CONTAINING DPP-4 INHIBITOR**
ZUSAMMENSETZUNG ZUR PRÄVENTION ODER BEHANDLUNG VON KLAPPENVERKALKUNG MIT DPP-4-INHIBITOR
COMPOSITION POUR LA PRÉVENTION OU LE TRAITEMENT DE LA CALCIFICATION DE VALVULE ET CONTENANT UN INHIBITEUR DE DPP-4

(43) Date of publication of application: 03.01.2018
(73) Proprietor: The Asan Foundation, Seoul 138-736 (KR); University Of Ulsan Foundation For Industry Cooperation, Ulsan 44610 (KR)
(72) Inventor: CHANG, Eun-Ju, Seoul 138-911 (KR); SONG, Jae-Kwan, Seoul 138-930 (KR); KIM, Mi Jeong, Incheon 403-720 (KR); CHOI, Bongkun, Gyeonggi-do 16903 (KR); LEE, Sahmin, Seoul 06012 (KR)
(74) Representative: dompatent von Kreisler Selting Werner - Partnerschaft von Patent- und Rechtsanwälten mbB
(86) International application number: PCT/KR2015/001902
(87) International publication number: WO 2016/137037

(56) References cited:
- KR-A- 20110 115 582
- KR-A- 20150 025 435
- BONGKUN CHOI ET AL: "Dipeptidyl Peptidase-4 Induces Aortic Valve Calcification by Inhibiting Insulin-Like Growth Factor-1 Signaling in Valvular Interstitial Cells", CIRCULATION, AMERICAN HEART ASSOCIATION, INC, US, vol. 135, no. 20, 16 May 2017 (2017-05-16) , pages 1935-1950, XP002781744, ISSN: 1524-4539, DOI: 10.1161/CIRCULATIONAHA.116.024270 [retrieved on 2017-02-08]
- SOON-YOUN CHOI ET AL: "Dipeptidyl peptidase-4 inhibitor gemigliptin protects against vascular calcification in an experimental chronic kidney disease and vascular smooth muscle cells", PLOS ONE, vol. 12, no. 7, 7 July 2017 (2017-07-07), page e0180393, XP055501362, DOI: 10.1371/journal.pone.0180393
- CHAYKOVSKA, L. ET AL.: 'Effects of DPP-4 Inhibitors on the Heart in a Rat Model of Uremic Cardiomyopathy' PLOS ONE vol. 6, no. 11, 01 November 2011, pages 1 - 9, XP055073329 DOI: 10.1371/JOURNAL.PONE.0027861
- SCHERNTHANER, G. ET AL.: 'Diabetic Nephropathy: New Approaches for Improving Glycemic Control and Reducing Risk' J NEPHROL vol. 26, no. 6, 01 January 2013, pages 975 - 985, XP055464099 DOI: 10.5301/JN.5000281
- LEE, DAE HO: 'Dipeptidyl Peptidase-4 Inhibitor' KOREAN JOURNAL OF INTERNAL MEDICINE vol. 87, no. 1, 01 January 2014, pages 1 - 8, XP055464100 DOI: 10.3904/KJM.2014.87.1.1
- SINGH MAHIPAL ET AL: "Osteopontin: At the cross-roads of myocyte survival and myocardial function", LIFE SCIENCES, PERGAMON PRESS, OXFORD, GB, vol. 118, no. 1, 28 September 2014 (2014-09-28), pages 1-6, XP029094699, ISSN: 0024-3205, DOI: 10.1016/J.LFS.2014.09.014
- SHAVELLE D M ET AL: "HMG CoA reductase inhibitor (statin) and aortic valve calcium", THE LANCET, ELSEVIER, AMSTERDAM, NL, vol. 359, no. 9312, 30 March 2002 (2002-03-30), pages 1125-1126, XP004790446, ISSN: 0140-6736, DOI: 10.1016/S0140-6736(02)08161-8

<110> THE ASAN FOUNDATION
<120> Composition for preventing or treating valvular calcification comprising Dipeptidyl peptidase-4 inhibitor
<130> PP-B1553
<160> 2
<170> KopatentIn 2.0
<210> 1
   <211> 32
   <212> DNA
   <213> Artificial Sequence
<220>
   <223> primer
<400> 1
   cagccagata acatgccaca cccaacccct tc 32
<210> 2
   <211> 32
   <212> DNA
   <213> Artificial Sequence
<220>
   <223> primer
<400> 2
   gaaggggttg ggtgtggcat gttatctggc tg 32

## Description

### TECHNICAL FIELD

The present disclosure relates to a pharmaceutical composition for use in prevention or treatment of aortic valvular calcification, which includes a dipeptidyl peptidase-4 (DPP-4) inhibitor.

### BACKGROUND ART

Cardiovascular calcification contributes to exacerbation of hypertension, heart failure, acute coronary syndrome, and valvular diseases, and thus causes various complications. Also, a variety of epidemiological studies have demonstrated that vascular calcification independently increases the mortalities. Vascular calcification occurs in a mechanism similar to an osteogenic program after a normal prenatal stage or a bone fracture, and is activated in diseases such as senility, diabetes, chronic renal failure, and chronic inflammatory diseases. The loss of minerals in bones is accelerated by an inflammatory response, and the free minerals are endocytosed into abnormal vascular endothelial cells.

Vascular endothelial dysfunction is an important mechanism for vascular calcification. Normally, endothelial nitric oxide synthase (eNOS) is constitutionally expressed at a certain level in vascular endothelial cells, endomyocardial cells, atrial cells, vascular smooth muscle cells, respiratory endothelial cells, and the like, and serves to adjust a vascular tone and maintain homeostasis of vascular endothelial cells by forming nitric oxide (NO). When the dysfunction of eNOS appears, isoenzymes, such as neuronal NOS (nNOS) or inducible NOS (iNOS), which have structures and functions similar to the eNOS, increase compensatorily to replace the role of eNOS, but do not continuously maintain normal vascular endothelial cell functions with ease. Therefore, pathologic alterations in blood vessels including atherosclerosis occur at an early stage. In a laboratory animal model studying an effect of endothelial nitric oxide synthase (eNOS) on the cardiovascular system, that is, an endothelial nitric oxide synthase knockout (eNOS KO) animal model, it was known that the onset of hypertension and atherosclerosis increases, and wide lesional areas and severe remodeling occur after apoplexy and myocardial infarction, compared to the control.

Meanwhile, although it has been reported that DPP-4 is expressed at an increased level in an animal model in which the cardiovascular calcification is induced, it has not been suggested that the expression of DPP-4 is associated with the calcification of the cardiovascular system, particularly heart valves, or the inhibition of expression or activity of DPP-4 can prevent or treat the calcification of heart valves.

Shavelle, D.M., Takasu, J., Budoff, M.J., Mao, S., Zhao, X.Q. and D O'Brien, K., 2002. HMG CoA reductase inhibitor (statin) and aortic valve calcium, The Lancet, 359(9312), pp.1125-1126, discloses 3-hydroxy-3-methylglutaryl-coenzyme A (HMG COA) reduces inhibitors (statins) for use in the treatment of aortic valvular calcification. Chaykovska, L., von Websky, K., Rahnenführer, J., Alter, M., Heiden, S., Fuchs, H., Runge, F., Klein, T. and Hocher, B., 2011. Effects of DPP-4 inhibitors on the heart in a rat model of uremic cardiomyopathy. PloS one, 6(11), discloses that DPP-4 inhibitors decrease the plasma concerntration of the vascular calcification marker, osteopontin.

The present inventors have made extensive efforts to elucidate a cardiovascular calcification progression and develop a therapeutic drug for cardiovascular calcification, and as a result, have found that a DPP-4 inhibitor can effectively prevent or treat calcification of valves, particularly heart valves, thereby completing the present invention.

### DISCLOSURE OF INVENTION

The present disclosure provides a pharmaceutical composition for use in prevention or treatment of aortic valvular calcification, which includes a dipeptidyl peptidase-4 (DPP-4) inhibitor. The embodiment of the present invention is reflected in independent claim 1. The preferred embodiments of the present invention are reflected in dependent claims 2 to 4.

### BRIEF DESCRIPTION OF THE DRAWINGS

FIG. 1 shows expression profiles of calcification-related genes, fibrosis-related genes and inflammation-related genes in CAVD patients. According to FIG. 1, up-regulation is indicated by red color, and down-regulation is indicated by green color.
FIG. 2a shows hDPP-4 mRNA expression levels in CAVD patients and normal persons, presented by qRT-PCR; FIG. 2b shows expression profiles of chemokines activated by DPP-4 and genes regulated by DPP-4; and FIG. 2c shows the results of qRT-PCR performed to measure the mRNA levels of genes regulated by DPP-4 in a microarray set.
FIG. 3 shows the results of histological analysis performed by von Kossa staining, Alizarin red staining, DAPI, DPP-4 and a merge of DAPI and DPP-4 in CAVD patients and normal persons. The nucleus was stained with DAPI (blue).
FIG. 4 shows the results of AR and VK staining of paraffin-embedded sections from wild-type or eNOS-/- mouse aortic valves. The sections were also stained with anti-murine DPP-4.
FIG. 5 shows the results of ALP, AR and VK staining of wild-type or eNOS-/- mouse VSMCs after incubation in osteogenic basal medium.
FIG. 6a shows the results of measuring the mDPP-4 levels of wild-type or eNOS-/- mouse VSMCs in culture medium by ELISA; FIG. 6b shows the results of analyzing mDPP-4 mRNA expression levels by qRT-PCR; and FIG. 6c shows the results of measuring plasma mDPP-4 levels by ELISA.

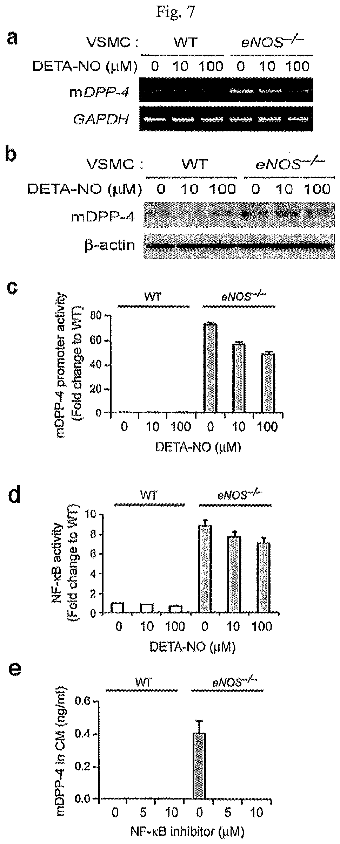

FIG. 7a shows the results of analyzing DPP-4 expression in wild-type or eNOS-/- mouse VSMCs by RT-PCR after 24 hours of treatment with DETA-NO; FIG. 7b shows the results of analyzing mDPP-4 expression in wild-type or eNOS-/- mouse VSMCs by immunoblot analysis after 48 hours of treatment with DETA-NO; FIG. 7c shows the results of measuring mDPP-4 promoter activity in wild-type or eNOS-/- mouse VSMCs; FIG. 7d shows the results of analyzing NF-κB activity in wild-type or eNOS-/- mouse VSMCs; and FIG. 7e shows the results of measuring mDPP-4 levels in wild-type or eNOS-/- mouse VSMCs, treated with a NF-κB inhibitor, by ELISA.
FIG. 8 shows the results of measuring mDPP-4 promoter activity in eNOS-/- mouse VSMCs, transformed with an mDPP-4 promoter containing an NF-κB binding site and with a promoter containing the luciferase gene, after treatment with DETA-NO.
FIGS. 9a and 9b show the results of administering a bisphosphonate-conjugated imaging agent to detect osteogenic activity, after administering sitagliptin to eNOS-/- mice.
FIG. 10a shows the results of VK staining of the whole body (upper raw) and aortic valve (lower raw) of eNOS-/- mice after administration of sitagliptin to the eNOS-/- mice; and FIG. 10b graphically shows a VK positive area.
FIG. 11 shows that osteoblast differentiation of human valvular interstitial cells (hVICs) is inhibited by treatment with DPP-4.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods, which will be described below, are those well known and commonly employed in the art.

The present disclosure is directed to a composition for use in prevention and treatment of valvular calcification, which includes a DPP-4 (Dipeptidyl peptidase-4) inhibitor.

In the present disclosure, the DPP-4 (Dipeptidyl peptidase-4) is also designated as a cluster of differentiation 26 (CD26), and is known as a protein involved in immunomodulation, apoptosis, signal transduction, and the like.

In the composition of the present disclosure, the DPP-4 inhibitor may be an inhibitor for inhibiting the activity of a DPP-4 protein.

In addition, examples of the inhibitor for inhibiting the activity of the DPP-4 protein may include an antibody against DPP-4, sitagliptin, vildagliptin, saxagliptin, linagliptin, dutogliptin, gemigliptin, alogliptin, anagliptin, evogliptin, berberine, diprotin, or lupeol. In the present disclosure, the antibody against the DPP-4 may be either a monoclonal antibody or a polyclonal antibody.

In the present disclosure, the level of DPP-4 expression is increased when valves are calcified, and the calcification decreases remarkably upon administration of the DPP-4 inhibitor. Therefore, the DPP-4 inhibitor can be useful in treatment or prophylaxis of the valves.

In the present disclosure, the valvular calcification refers to formation, growth or deposition of extracellular matrix hydroxyapatite (calcium phosphate) crystal deposits in the valves.

The calcification includes medial calcification, or atherosclerotic calcification. A calcified tissue is referred to as a calcified cartilage. The valvular calcification occurs characteristically at two regions. Intimal calcification occurs in connection with atherosclerosis. In the case of atherosclerosis, fat-rich macrophagocytes and T lymphocytes are first accumulated in valves to form a layer of fat, and smooth muscle cells then migrate from the media into the layer of fat. Chemokinetic compounds stimulating the movement of such cells are considered to be produced in proximal endothelial cells, activated phagocytes, and the like. The migrated smooth muscle cells proliferate, fats are accumulated in the smooth muscle cells, and extracellular matrices are formed. The calcification occurs at the central region of an artheromatous plaque. The medial calcification occurs regardless of atherosclerosis and intimal calcification. The medial arterial calcification occurring at the distal arteries is also referred to as Monckeberg's sclerosis, and is often observed in the aged diabetic patients. It is known that smooth muscle cells and elastin are associated with the occurrence of the medial arterial calcification.

As used herein, the term "atherosclerotic calcification" refers to vascular calcification occurring in artheromatous plaques along the intima.

In the present disclosure, the medial calcification, the medial wall calcification, or the Monckeberg's sclerosis refers to calcification characterized by the presence of calcium in the medial wall.

Also, the calcification according to the present disclosure may be caused by a valvular disease, hyperlipidaemia, senility, estrogen deficiency, angina, heart failure, nephritis, uremia, diabetes, an inflammatory disease, or a cardiovascular disorder. Examples of the nephritis may include glomerulonephritis, diabetic nephritis, lupus nephritis, multiple pustular nephritis, pyelonephritis, lithonephria, nephrotuberculosis, renal tumor, and the like. Also, examples of the inflammatory disease may include asthma, allergic and non-allergic rhinitis, chronic and acute rhinitis, chronic and acute gastritis or enteritis, ulcerative gastritis, acute and chronic nephritis, acute and chronic hepatitis, a chronic obstructive pulmonary disease, pulmonary fibrosis, an irritable bowel syndrome, inflammatory pain, migraine headache, headache, backache, a fibromyalgia syndrome, a myofascial disease, a viral infection (for example, hepatitis C), a bacterial infection, a fungal infection, a burn, an injury caused by a surgical or dental operation, a prostaglandin E overload syndrome, atherosclerosis, gout, arthritis, rheumatic arthritis, ankylosing spondylitis, a Hodgkin's disease, pancreatitis, conjunctivitis, iritis, scleratitis, uveitis, dermatitis, eczema, and multiple sclerosis. In addition, examples of the cardiovascular disease may include myocardiopathy, a primary cardiac arrest, ischemic heart failure, hypertension, an ischemic heart disease, a coronary artery disease, angina, myocardial infarction, atherosclerosis, and arrhythmia.

In the present disclosure, it was found that DPP-4 is involved in the proceeding process of an aortic valvular calcification (CAVD) in the aortic valvular calcification. A deficiency of NO in a mouse lacking the supply of NO increases the expression of DPP-4. This will cause osteoblast metastasis and differentiation and result in an acceleration of calcification in the aortic valve.

When a DPP-4 inhibitor is administered to a subject, the osteoblast metastasis of VSMC can be inhibited to inhibit the progression of diseases, which supports a concept that DPP-4 can be targeted for the potential treatment in the treatment of CAVD.

The pharmaceutical composition according to one exemplary embodiment of the present disclosure may further include a pharmaceutically acceptable carrier. The pharmaceutically acceptable carrier may include a saline solution, sterile water, a Ringer's solution, a buffered saline solution, dextrose solution, maltodextrin solution, glycerol, ethanol and a mixture of two or more thereof, which may be used alone or in combination. When necessary, other conventional additives such as an antioxidant, a buffer and a bacteristat may be added to the composition. Also, a diluent, a dispersing agent, a surfactant, a binding agent and a lubricating agent may be further added to the composition. In this case, the composition may be formulated into an injectable formulation such as an aqueous solution, a suspension, or an emulsion, a pill, a capsule, a granule, or a tablet. In addition, the pharmaceutical composition may be preferably formulated according to individual diseases and components using a proper method known in the related art, or a method disclosed in Remington's Pharmaceutical Science (recent Version), Mack Publishing Company, Easton Pa. Examples of the pharmaceutically acceptable carriers, the formulations of the pharmaceutical composition, and methods of preparing the formulations are known in the related art.

Also, such pharmaceutical compositions may be useful in administering a composition including the DPP-4 inhibitor of the present disclosure as an active ingredient to a subject so as to treat the valvular calcification, as described above. The composition according to the present disclosure may be provided at an effective amount to treat the valvular calcification in the subject in need. The dose of the DPP-4 inhibitor may vary to a wide extent according to a patient's weight, age, gender, health condition, diet, administration time, administration method, excretion rate, and severity of a disease. In this case, the proper dose of the DPP-4 inhibitor may be optionally determined by those skilled in the related art.

A daily dosage of the DPP-4 inhibitor is about 0.0001 to 500 mg/kg, preferably about 0.01 to 5 mg/kg, and may be administered once or in divided doses a day.

The composition of the present disclosure may be administered orally, or parenterally (for example, intravenously, subcutaneously, intraperitoneally, intramuscularly, intravascularly, or subcutaneously) according to a desired purpose. Preferably, the composition may be administered orally. For oral administration, the composition may be provided in the form of a capsule, a tablet, a powder, a granule, or a suspension. Also, the formulation may include a conventional additive such as lactose, mannitol, corn starch, or potato starch, and the binding agent that can be used in such formulations may include crystalline cellulose, cellulose analogue, acacia, corn starch, or sodium carboxymethyl cellulose.

In addition, the formulations may be provided with dibasic calcium phosphate, or anhydrous sodium starch glycolate. Finally, the formulations may be provided with a lubricating agent, for example, talc or magnesium stearate.

Further, the composition of the present disclosure may be used alone or in combination with surgery, hormone treatment, drug treatment, and methods using a biological response modifier in order to prevent or treat the valvular calcification.

### EXAMPLES

Hereinafter, the present disclosure will be described in further detail with reference to examples. It will be obvious to a person having ordinary skill in the art that these examples are illustrative purposes only and are not to be construed to limit the scope of the present disclosure.

### Example 1: Analysis of DPP-4 Expression in Human Calcific Aortic Valve Disease (CAVD)

Human aortic valve samples were obtained from normal patients who received aortic valve replacement in Seoul Asan Medical Center and from patients with severe calcified lesions. Half of each sample was immediately stored in RNAlater (Life Technology), and the remaining half was fixed in an OCT compound (Sakura Finetek, Inc.). The study protocol for collection of human samples was approved by the Institutional Review Board (IRB) of Seoul Asan Medical Center (Seoul, Korea).

Gene expression profiles that are involved in cell progression in human calcific aortic valve disease (CAVD) were analyzed by microarray.

Analysis of gene expression profiles in aortic valve samples from non-calcified normal patients and calcified patients was performed using an Affymetrix GeneChip® Human Gene 2.0 ST array (Affymetrix, USA). Q uantile normalization and data analysis were performed using Affymetrix GCOS software (Affymetrix, Inc.).

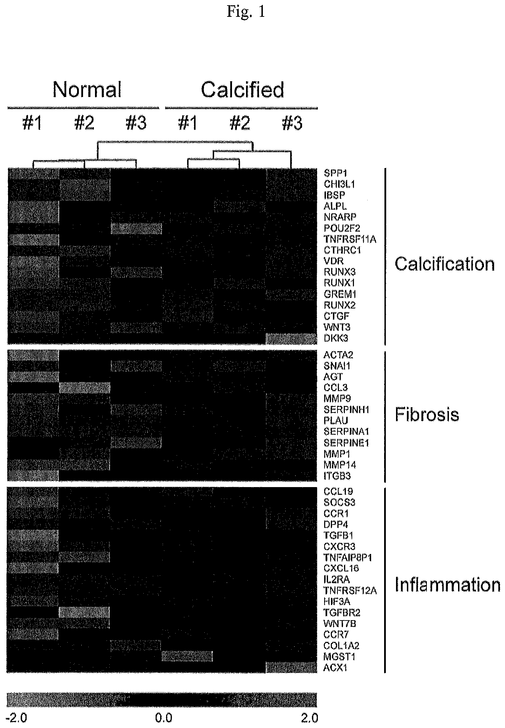

As a result, as shown in FIG. 1, genes related to calcification, fibrosis and inflammation were up-regulated in the aortic valves of CAVD patients compared to normal persons.

Furthermore, real-time PCR (qRT-PCR) was performed to measure DPP-4 expression in CAVD patients. In the qRT-PCR process, total RNA was isolated from each sample by use of a RNeasy Lipid Tissue kit (Qiagen, USA), and synthesized into cDNA by use of a cDNA synthesis kit (Thermo scientifir, EU). qRT-PCR analysis of the synthesized cDNA was performed in an optical 96-well plate by use of a Power SYBR green 1step kit and an ABI 7000 real-time PCR system (Applied Biosystems). As an internal control, GAPDH was used.

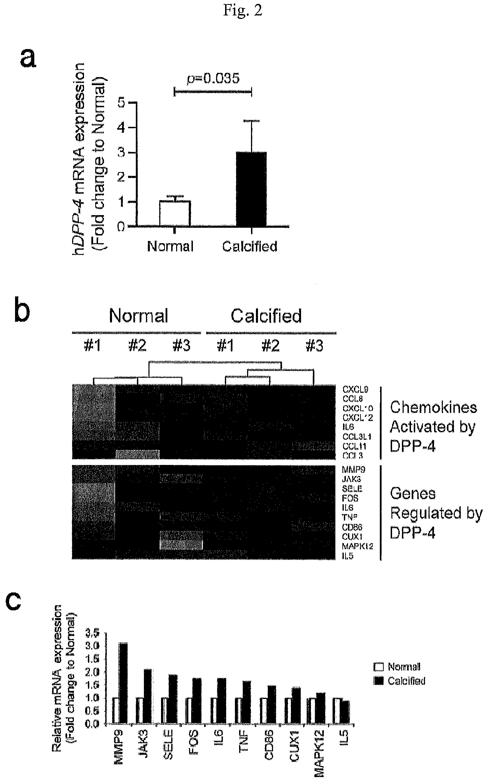

As a result, as shown in FIG. 2a, the results of real-time PCR indicated that DPP-4 was expressed at high levels in CAVD patients.

The results of microarray analysis indicated that DPP-4 activity activated inflammatory chemokines and that downstream genes regulated by DPP-4 and chemokine genes were up-regulated in calcified aortic valve tissue (FIG. 2b). In addition, up-regulation of the genes was confirmed again by analyzing mRNA expression using real-time PCR (FIG. 2c).

Human aortic valve samples were fixed in 4% formalin solution for 24 hours, and then embedded in paraffin and sectioned to a thickness of 4 µm. In order to observe calcium deposition in the paraffin-embedded aortic valve and the cardiac basal segment, Von Kossa (VK) staining and Alizarin red (AR) staining were performed. For Von Kossa staining, the aortic valve fixed in formalin was washed with distilled water, and then exposed to 5% water-soluble AgNO₃ and intense light at room temperature for 60 minutes. Next, the aortic valve was treated with 2.5% sodium thiosulfate for 5 minutes, and when it showed blackish brown color, it was regarded as positive. For Alizarin red staining, the aortic valve fixed in formalin was washed with distilled water, and then treated with 2% Alizarin red S (aqueous, Sigma) for 5 minutes, and when the aortic valve showed red or orange color, it was regarded as positive.

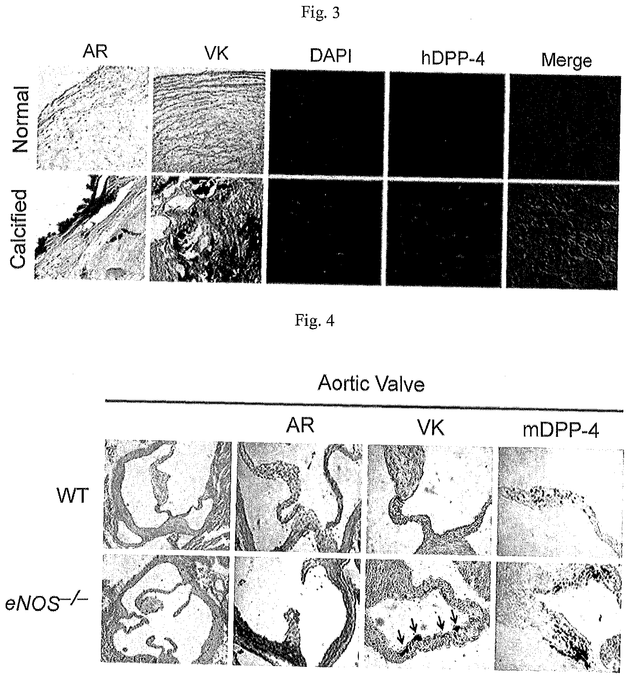

Immunofluorescence staining of the calcified aortic valve tissue was performed. As a result, as shown in FIG. 3, DPP-4 was highly expressed in a calcified portion stained with Von Kossa (VK) and Alizarin red, and expression of DPP-4 was not observed in the aortic valve tissue of normal persons.

### Example 2: Analysis of DPP-4 Expression in eNOS Knockout Mouse Models

C57BL/6J eNOS-/- (endothelial nitric oxide synthase knockout) mice (8-week old, n = 10, The Jackson Laboratory, Bar Harbor, Me, USA) and C57BL/6J wild-type mice (8-week old, wide type, WT, n = 10) were used. After purchase, the mice were fed with standard rodent feed up to 3 months of age.

The mice were deeply anesthetized, and in this state, PBS containing 4% paraformaldehyde was administered to the mice, and the heart and the aorta were extracted. The extracted tissues were fixed overnight and embedded in paraffin, and the entire aortic valve was sectioned to a thickness of 5 µm. The mice were euthanized, and the blood and aorta samples were stored for analysis.

To observe calcium deposition in the paraffin-embedded aortic valve and the cardiac basal segment, von Kossa staining and Alizarin red staining were performed.

As a result, as shown in FIG. 4, a blackish brown stained portion indicating mineralization in the aortic valve of the eNOS KO mouse was observed in von Kossa staining of the aortic valve, unlike the control, and a red stained portion indicating calcium deposition was also observed in Alizarin red staining of the aortic valve. Furthermore, in AR staining and VK staining, cells that expressed a high level of DPP-4 were observed in a calcified region.

### Example 3: Observation of Calcification in Mouse Vascular Smooth Muscle Cells (VSMC)

The aortas, extracted from the eNOS-/- mice and the wild-type mice together with the hearts in Example 2, were washed in serum-free M199 (Cellgro), cut to small pieces, and then cultured in M199 containing 20% fetal calf serum and 3 mg/mL collagenase (Sigma) in a water bath at 37°C for 3 hours. The isolated cells were found to be vascular smooth muscle cells by use of anti-SM monoclonal antibody (Sigma). The isolated vascular smooth muscle cells were seeded at a concentration of 30%, and after 24 hours, the medium was replaced with osteogenic basal medium (Osteogenic SingleQuots, Lonza, USA). The medium was replaced at 3-day intervals during 28 days of culture.

For the wild-type mice and eNOS KO mice, it was determined whether CD26 would promote osteogenic differentiation of vascular smooth muscle cells, by use of ALP staining as a staining method for the initial calcification stage, Alizarin red (AR) staining as a staining method for the middle calcification stage, and von Kossa (VK) staining as a staining method for the late calcification stage.

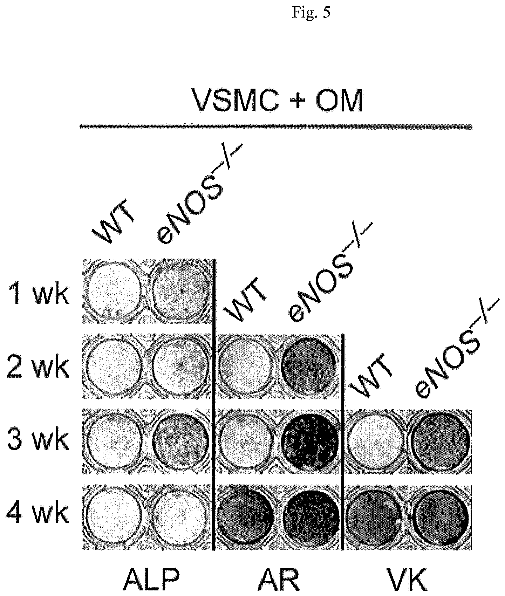

As a result, as can be seen in FIG. 5, osteogenic differentiation of vascular smooth muscle cells (VSMCs) was promoted in the eNOS knockout transgenic mice compared to the control mice.

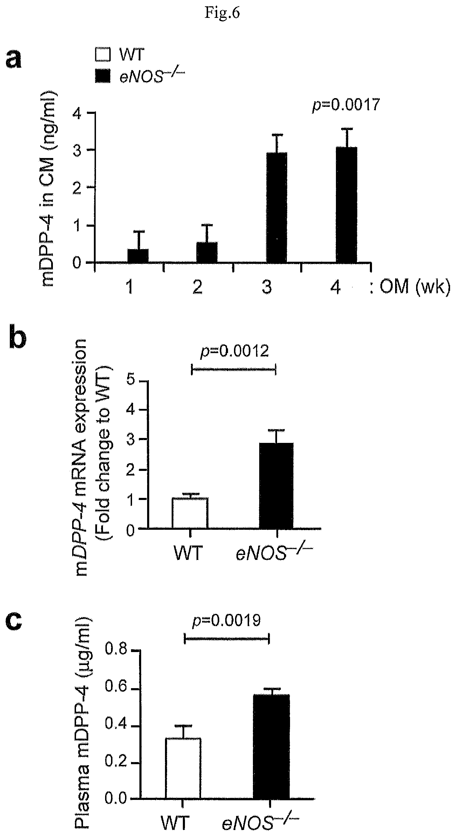

The concentration of free CD26/DPP-4 in a culture of the vascular smooth muscle cells was measured using a commercial ELISA kit (Sigma) according to the manufacturer's instructions. As a result, as shown in FIG. 6a, increased DPP-4 concentration in a culture of VSMCs from the eNOS -/- mice.

DDP-4 mRNA expression levels in the aortic valve tissues were measured by real-time PCR. As a result, as could be seen in FIG. 6b, DPP-4 mRNA expression increased in the aortic valve tissue of the eNOS -/- mice compared to that of the wild-type mice.

In addition, plasma DPP-4 protein levels in the eNOS -/mice and the wild-type mice were measured by ELISA. As a result, as could be seen in FIG. 6c, the plasma DPP-4 protein level increased in the eNOS -/- mice compared to the wild-type mice.

### Example 4: Observation of NO-Induced Regulation of DPP-4 Expression in Vascular Smooth Muscle Cells (VSMCs)

Treatment with L-NAME (Nω-nitro-L-arginine methyl ester) that an NO biosynthesis inhibitor significantly increases DPP-4 expression in the aorta. Thus, it is known that NO depletion induces DDP-4 expression.

In this Example, vascular smooth muscle cells obtained according to the method of Example 3 were treated with DETA-NONOate (DETA-NO) that is an NO donor. As a result, it was shown that expression of DPP-4 mRNA in the VSMCs decreased (FIG. 7a) and expression of DPP-4 protein in the VSMCs also decreased (FIG. 7b).

To obtain more direct evidence for the regulatory function of NO in a decrease in DPP-4 expression, it was determined whether DPP-4 promoter activity would be regulated by NO.

As a result, it was shown that the DPP-4 promoter activity was higher in eNOS-/- mouse VSMCs than in wild-type mouse VSMCs, and this activity was inhibited by treatment with DETA-NO in a concentration-dependent manner (FIG. 7c).

NF-κB activity in VSMCs is inhibited by NO. It was shown that NF-κB activity was higher in eNOS-/- mouse VSMCs than in wild-type mouse VSMCs, and this activity was reduced by treatment with DETA-NO (FIG. 7d). Because the DPP4 promoter has a putative NF-κB binding site, the present inventors determined whether NF-κB activity could directly regulate DPP-4 promoter activity.

First, VSMCs were cultured in a 6-well plate to reach a confluency of 80%. Using Lipofectamine2000 (Life Technology), a DPP-4 reporter containing a normal or mutated NF-κB binding site was cloned into a pGL3-basic vector (Promega), and the cells were transfected with the vector. After 24 hours, firefly and Renilla luciferase activities in the cells were measured using a Dual-Glo luciferase assay system, (Promega). The firefly luciferase activity was normalized with the Renilla luciferase activity.

For site-directed mutagenesis (SAM) of the NF-κB binding site, PCR was performed using the following primers.
SEQ ID NO 1: 5'-CAGCCAGATAACATGCCACACCCAACCCCTTC-3;
SEQ ID NO 2: 5'-GAAGGGGTTGGGTGTGGCATGTTATCTGGCTG-3'.

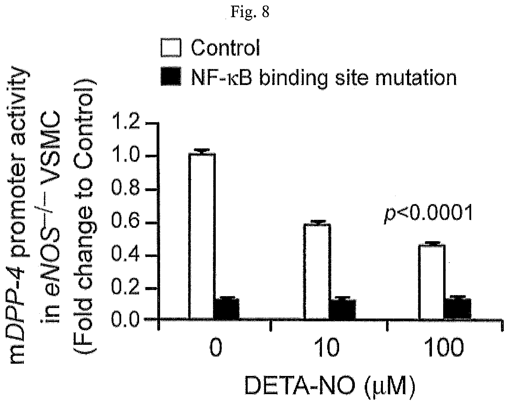

As a result, as shown in FIG. 8, mutation of the NF-κB binding site of the DPP-4 promoter significantly inhibited the DPP-4 promoter activity, and treatment with the NF-κB inhibitor completely inhibited DPP-4 production (FIG. 7e). Such results suggest that induction of DPP-4 by NF-κB activation in vascular smooth muscle cells occurs by NO depletion and that osteogenic differentiation of VSMCs can be regulated by controlling NO supply.

### Example 5: Evaluation of the Effect of DPP-4 Inhibitor Sitagliptin on Aortic Valve Calcification

A significant increase in DPP-4 during aortic valve calcification suggests that DPP-4 plays a potential role in aortic valve calcification. To demonstrate suggestion, aortic calcification of eNOS-/- mice was treated with the selective DPP-4 inhibitor sitagliptin, and the effect of sitagliptin was examined.

C57BL/6J eNOS-/- (endothelial nitric oxide synthase knockout) mice (8-week old, n = 10, The Jackson Laboratory, Bar Harbor, Me, USA) and C57BL/6J wild-type mice (8-week old, wide type, WT, n = 10) were used. After purchase, the mice were fed with standard rodent feed up to 3 months of age, and administered with saline or sitagliptin (15 mg/kg/ day, Merck & Co., Inc.) for 12 weeks.

To detect osteogenic activity in the eNOS-/- mice, a bisphosphonate-conjugated imaging agent (Osteosense680, VisEn Medical Inc.) was injected via the tail vein. Osteosense680 binds to a calcified site (particularly hydroxyapatite) *in vivo* and acts as an imaging agent to detect osteoblast activity. The mice were euthanized, and then the whole body or isolated aortas were imaged using OptixMX3 (ART Advanced Research Technologies, Inc).

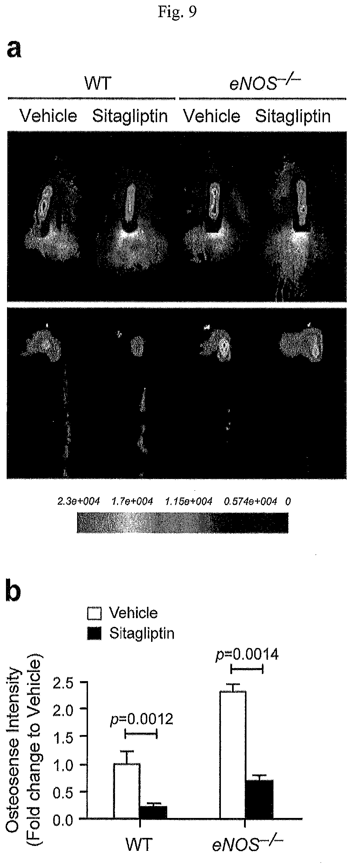

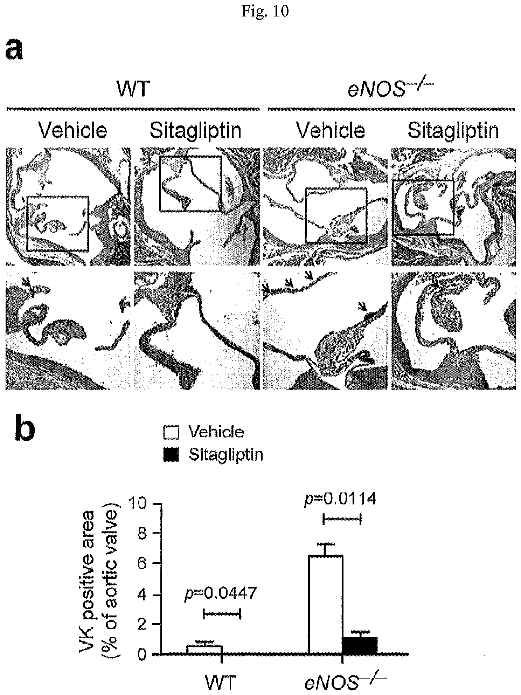

As a result, it was shown that there was severe aortic calcification in the eNOS-/- mice (FIG. 9), and that oral administration of sitagliptin reduced aortic calcification in the living mice (FIGS. 9a and 9b). Furthermore, in VK-stained sections of the aortic valves from the eNOS-/- mice, it was found that when DPP-4 activity is inhibited, aortic calcification can be inhibited (FIGS. 10a and 10b).

### Example 6: Evaluation of the Effect of Sitagliptin Treatment in Human Aortic Valvular Interstitial Cells (VICs)

Human aortic valvular interstitial cells (VICs) are a collection of different kinds of cells, including fibroblasts, myoblasts and smooth muscle cells, have characteristics similar to those of VSMCs, and are capable of differentiating into osteoblasts.

Using human VICs, the role of DPP-4 in osteoblast differentiation was analyzed.

Human VICs were isolated from aortic valve leaflets according to a conventional cell isolation method, and VIC cells at passage 2 to 10 were used. The VIC cells were incubated with DPP-4 (R & D), sitagliptin or DETA-NONOate (Enzo Life Sciences).

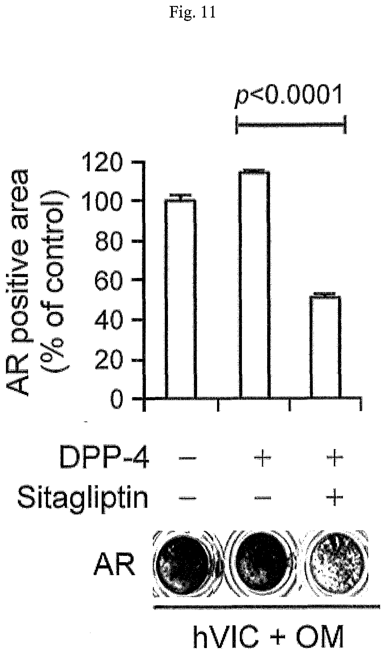

As a result, as shown in FIG. 11, DPP-4 treatment increased the osteogenic ability of VICs, and this phenomenon was eliminated by sitagliptin treatment.

To examine the relation of DPP-4 with disease activity, plasma DPP-4 levels in CAVD patients were analyzed. Patients with rheumatic aortic valve diseases and patients administered with a DPP-4 inhibitor were excluded from the analysis.

As a result, the DPP-4 level in the CAVD patients was higher than that in normal persons (Table 1).

**Table 1: Clinical characteristics of aortic valve disease patients**

| | Avpathology (-) (n=111) | AV pathology +) (n=48) | *p* value |
|---|---|---|---|
| Age, y^{a} | 60±41 | 78+15 | <0.001 |
| Male, n^{b} | 69 (62) | 20 (42) | 0.017 |
| Body surface area, m² | 1.7±0.4 | 1.54±0.35 | <0.001 |
| Systolic blood pressure, mmHg | 118±38 | 123±45 | 0.079 |
| Diastolic blood pressure, mmHg | 71+32 | 66±33 | 0.023 |
| Hypertension, n | 53 (48) | 37 (77) | 0.001 |
| Diabetes, n | 23 (21) | 12 (25) | 0.550 |
| Dyslipidemia, n | 53 (48) | 25 (52) | 0.616 |
| Renal insufficiency, n | 3 (3) | 8 (17) | 0.001 |
| Coronary artery disease, n | 56 (50) | 19 (40) | 0.208 |
| Previous stroke, n | 15 (14) | 9 (19) | 0.410 |
| Hemoglobin, g/dL | 13.6±4.5 | 11.8±6.7 | <0.001 |
| Creatinine, mg/dL | 0.95±6.26 | 1.48±7.76 | 0.006 |
| Cholesterol, mg/dL | 165±155 | 158±77 | 0.316 |
| Aortic Vmax (peak velocity), m/s | 1.4±1.1 | 4.6±2.1 | <0.001 |

The DPP-4 level was 34% higher in CAVD patients with calcification than in patients with no calcification, and DPP-4 having the ability to degrade activated protein also increased in the CAVD patients. This suggests that the DPP-4 circulation level is related to the severity of CAVD patients (Table 2).

**Table 21: Clinical characteristics of CAVD patients determined by CT calcium score**

| | Group I sevAS_AVCi (-) (n=50) | Group II sevAS_AVCi (+) (n=22) | *p* value |
|---|---|---|---|
| Age, y | 66±21 | 79±14 | <0.001 |
| Male, n | 34 (68) | 9 (41) | 0.031 |
| Body surface area, m² | 1.7±0.5 | 1.6±0.3 | 0.004 |
| Systolic blood pressure, mmHg | 120±45 | 121±41 | 0.789 |
| Diastolic blood pressure, mmHg | 71+32 | 62+28 | 0.004 |
| Hypertension, n | 31 (62) | 19 (86) | 0.039 |
| Diabetes, n | 13 (26) | 6 (27) | 0.910 |
| Dyslipidemia, n | 26 (52) | 14 (64) | 0.360 |
| Renal insufficiency, n | 2 (4) | 3 (14) | 0.138 |
| Coronary artery disease, n | 31 (62) | 7 (32) | 0.018 |
| Previous stroke, n | 9 (18) | 2 (9) | 0.318 |
| Hemoglobin, g/dL | 13.8±6.2 | 11.4±7.1 | <0.001 |
| Creatinine, mg/dL | 1.1±8.0 | 1.1±1.2 | 0.994 |
| Cholesterol, mg/dL | 167±129 | 157±66 | 0.262 |
| Agatston scorea (AU)^{a} | 230±832 | 611±2515 | 0.011 |
| Valve Calcium (AU)^{a} | 184±1386 | 3460±4282 | <0.001 |
| Total Calcium (AU)^{a} | 422±1878 | 4185±6464 | <0.001 |
| Valve calcium score/ BSA (AVCi, AU/ m²)^{a} | 119±866 | 2176±1964 | <0.001 |
| Aortic Vmax (peak velocity), m/s | 1.9±3.0 | 5.2±1.4 | <0.001 |

### Example 7: Evaluation of the Effects of Administration of Various DPP-4 Inhibitors on Inhibition of Osteoblast Differentiation of VSMCs

In order to examine the effects of DPP-4 inhibitors other than sitagliptin on the inhibition of valvular calcification, the effects of treatment with various kinds of DPP-4 inhibitors on the inhibition of osteogenic differentiation of eNOS-/- mouse VSMCs (vascular smooth muscle cells) were evaluated.

eNOS-/- mouse VSMCs cultured in a 48-well plate were treated with 10M of each of alogliptin, evogliptin, linaglipin, saxagliptin and vildagliptin for 7 days, and ALP activity in the cells was measured. As a result, ALP activity inhibition of 50% or lower compared to that in a negative control group not treated with the DPP-4 inhibitor was observed. This suggests that DPP-4 inhibitors other than sitagliptin also exhibit an inhibitory effect against aortic valvular calcification.

The substantial scope of the present disclosure will be defined by the appended claims.

### INDUSTRIAL APPLICABILITY

The DPP-4 of the present disclosure is expressed at an increased level when valves are calcified, and the calcification decreases remarkably upon administration of the DPP-4 inhibitor. Therefore, the DPP-4 inhibitor can be useful in treatment or prophylaxis of the valves.
## Claims

1. A pharmaceutical composition for use in preventing or treating aortic valvular calcification comprising a dipeptidyl peptidase-4 (DPP-4) inhibitor.

2. The pharmaceutical composition for use of claim 1, wherein the DPP-4 inhibitor is an inhibitor for inhibiting activity of a DPP-4 protein.

3. The pharmaceutical composition for use of claim 2, wherein the inhibitor for inhibiting the activity of the DPP-4 protein is at least one selected from the group consisting of an antibody against DPP-4, sitagliptin, vildagliptin, saxagliptin, linagliptin, dutogliptin, gemigliptin, alogliptin, anagliptin, evogliptin, berberine, diprotin, and lupeol.

4. The pharmaceutical composition for use of claim 1, wherein the aortic valvular calcification is caused by a cardiovascular disease, preferably at least one selected from the group consisting of myocardiopathy, a primary cardiac arrest, ischemic heart failure, hypertension, an ischemic heart disease, a coronary artery disease, angina, myocardial infarction, atherosclerosis, and arrhythmia.

## Patentansprüche

1. Pharmazeutische Zusammensetzung zur Verwendung bei der Prävention oder Behandlung einer Verkalkung der Aortenklappe, die einen Dipeptidyl-peptidase-4(DPP-4)-Inhibitor umfasst.

2. Pharmazeutische Zusammensetzung zur Verwendung gemäß Anspruch 1, wobei der DPP-4-Inhibitor ein Inhibitor zum Hemmen der Aktivität eines DPP-4-Proteins ist.

3. Pharmazeutische Zusammensetzung zur Verwendung gemäß Anspruch 2, wobei der Inhibitor zum Hemmen der Aktivität des DPP-4-Proteins wenigstens einer ist, der aus der Gruppe ausgewählt ist, die aus einem Antikörper gegen DPP-4, Sitagliptin, Vildagliptin, Saxagliptin, Linagliptin, Dutogliptin, Gemigliptin, Alogliptin, Anagliptin, Evogliptin, Berberin, Diprotin und Lupeol besteht.

4. Pharmazeutische Zusammensetzung zur Verwendung gemäß Anspruch 1, wobei die Verkalkung der Aortenklappe durch eine Herz-Kreislauf-Erkrankung verursacht ist, vorzugsweise wenigstens eine, die aus der Gruppe ausgewählt ist, die aus Myokardiopathie, primärem Herzstillstand, ischämischer Herzinsuffizienz, Bluthochdruck, ischämischer Herzerkrankung, koronarer Herzkrankheit, Angina pectoris, Myokardinfarkt, Atherosklerose und Arrhythmie besteht.

## Revendications

1. Composition pharmaceutique à utiliser dans la prévention ou dans le traitement d'une calcification de la valve aortique, comprenant un inhibiteur de la dipeptidyl peptidase-4 (DPP-4).

2. Composition pharmaceutique à utiliser selon la revendication 1, dans laquelle ledit inhibiteur de la DPP-4 est un inhibiteur pour inhiber l'activité d'une protéine DPP-4.

3. Composition pharmaceutique à utiliser selon la revendication 2, dans laquelle ledit inhibiteur pour inhiber l'activité de la protéine DPP-4 est au moins l'un choisi dans le groupe consistant en un anticorps contre la DPP-4, la sitagliptine, la vildagliptine, la saxagliptine, la linagliptine, la dutogliptine, la gémigliptine, l'alogliptine, l'anagliptine, l'évogliptine, la berbérine, la diprotine, et le lupéol.

4. Composition pharmaceutique à utiliser selon la revendication 1, dans laquelle la calcification de la valve aortique est causée par une maladie cardiovasculaire, de préférence au moins une choisie dans le groupe consistant en myocardiopathie, arrêt cardiaque primaire, insuffisance cardiaque ischémique, hypertension, cardiopathie ischémique, coronaropathie, angine de poitrine, infarctus du myocarde, athérosclérose, et arythmie cardiaque.
